(19) Europäisches Patentamt / European Patent Office / Office européen des brevets

(11) **EP 2 844 694 B1**

(12) **EUROPEAN PATENT SPECIFICATION**

(45) Date of publication and mention
of the grant of the patent:
**09.08.2017 Bulletin 2017/32**

(21) Application number: **13718747.2**

(22) Date of filing: **15.04.2013**

(51) Int Cl.:
***C08K 3/04*** *(2006.01)*

(86) International application number:
**PCT/US2013/036565**

(87) International publication number:
**WO 2013/165677 (07.11.2013 Gazette 2013/45)**

(54) **RUBBER FORMULATIONS INCLUDING GRAPHENIC CARBON PARTICLES**

KAUTSCHUKFORMULIERUNGEN MIT GRAPHENKOHLENSTOFFTEILCHEN

FORMULATIONS DE CAOUTCHOUC CONTENANT DES PARTICULES DE CARBONE GRAPHÉNIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2012 US 201213462955**

(43) Date of publication of application:
**11.03.2015 Bulletin 2015/11**

(73) Proprietor: **PPG Industries Ohio, Inc.
Cleveland, OH 44111 (US)**

(72) Inventors:
• **MARTIN, Justin J.**
**Jeannette, Pennsylvania 15644 (US)**
• **VANIER, Noel R.**
**Wexford, Pennsylvania 15090 (US)**
• **REARICK, Brian K.**
**Allison Park, Pennsylvania 15101 (US)**
• **KOLLAH, Raphael O.**
**Wexford, Pennsylvania 15090 (US)**
• **OKEL, Timothy A.**
**Trafford, Pennsylvania 15085 (US)**
• **ASAY, David**
**Sarver, Pennsylvania 16055 (US)**

• **KAHLE, Charles F.**
**Pittsburgh, Pennsylvania 15237 (US)**
• **HUNG, Cheng-Hung**
**Wexford, Pennsylvania 15090 (US)**

(74) Representative: **f & e patent
Fleischer, Engels & Partner mbB, Patentanwälte
Braunsberger Feld 29
51429 Bergisch Gladbach (DE)**

(56) References cited:
**WO-A1-2010/016976     US-A1- 2011 046 289**

• **DUNG-HO SONG, O-SEOK KWON, HO-KYUN HEONG, YONG-GU KANG: "Properties of Styrene-Butadiene Rubber Nanocomposites Reinforced with Carbon Black, Carbon Nanotube, Graphene, Graphite", KOR. J. MATER. RES., vol. 20, no. 3, 1 February 2010 (2010-02-01), pages 104-110, XP002711182, DOI: 10.3740/MRSK.2010.20.2.104**
• **DATABASE WPI Week 201074 Thomson Scientific, London, GB; AN 2010-L56434 XP002711183, & KR 2010 0096918 A (HYUNDAI MOTOR CO LTD) 2 September 2010 (2010-09-02)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to rubber formulations comprising graphenic carbon particles.

BACKGROUND OF THE INVENTION

Various fillers have been added to rubber compositions. For example, carbon black has been utilized in various parts of tires including the tread to reduce build up of electrical charge. In addition, silica has been utilized in tire treads to reduce rolling resistance. While it is desirable to add significant amounts of silica in order to improve the performance characteristics of tire tread formulations, the maximum amount that can be added is limited by the relatively large amount of carbon black that is added to adequately reduce the build up of electrical charge. Mechanical, electrical and thermal properties of rubber compositions reinforced with carbon materials have been disclosed by Yong-Gu Kang et. al. Kor. J. Meter. Res. 2010, 20, 104-110 and in KR 2010 0096918 A.

SUMMARY OF THE INVENTION

An aspect of the invention provides a rubber formulation comprising a base rubber composition, from 0.1 to 20 weight percent graphenic carbon particles, and from 1 to 50 weight percent filler particles, wherein the graphenic carbon particles have an oxygen content of less than 2 atomic weight percent and the tire tread formulation has a surface resistivity of less than $10^{10}$ Ω/sq.

Another aspect of the invention provides a method of making a rubber formulation comprising mixing graphenic carbon particles and filler particles with a base rubber composition, and curing the mixture, wherein the graphenic carbon particles have an oxygen content of less than 2 atomic weight percent and the cured mixture has a surface resistivity of less than $10^{10}$ Ω/sq.

DETAILED DESCRIPTION OF EMBODIMENTS OF THE INVENTION

Rubber formulations in accordance with embodiments of the present invention are useful in various applications including tire components such as vehicle tire treads, subtreads, tire carcasses, tire sidewalls, tire belt wedge, tire bead, and tire wire skim coat, wire and cable jacketing, hoses, gaskets and seals, industrial and automotive drive-belts, engine mounts, V-belts, conveyor belts, roller coatings, shoe sole materials, packing rings and damping elements. While tire tread formulations are described herein as a particular embodiment of the invention, it is to be understood that the rubber formulations of the present invention are not limited to such uses and may be used in various other applications.

The rubber formulations of the present invention comprise a base rubber composition to which graphenic carbon particles are added. As used herein, the term "graphenic carbon particles" means carbon particles having structures comprising one or more layers of one-atom-thick planar sheets of $sp^2$-bonded carbon atoms that are densely packed in a honeycomb crystal lattice. The average number of stacked layers may be less than 100, for example, less than 50. In certain embodiments, the average number of stacked layers is 30 or less, such as 20 or less, 10 or less, or, in some cases, 5 or less. The graphenic carbon particles may be substantially flat, however, at least a portion of the planar sheets may be substantially curved, curled, creased or buckled. The particles typically do not have a spheroidal or equiaxed morphology.

In certain embodiments, the graphenic carbon particles present in the compositions of the present invention have a thickness, measured in a direction perpendicular to the carbon atom layers, of no more than 10 nanometers, no more than 5 nanometers, or, in certain embodiments, no more than 4 or 3 or 2 or 1 nanometers, such as no more than 3.6 nanometers. In certain embodiments, the graphenic carbon particles may be from 1 atom layer up to 3, 6, 9, 12, 20 or 30 atom layers thick, or more. In certain embodiments, the graphenic carbon particles present in the compositions of the present invention have a width and length, measured in a direction parallel to the carbon atoms layers, of at least 50 nanometers, such as more than 100 nanometers, in some cases more than 100 nanometers up to 500 nanometers, or more than 100 nanometers up to 200 nanometers. The graphenic carbon particles may be provided in the form of ultrathin flakes, platelets or sheets having relatively high aspect ratios (aspect ratio being defined as the ratio of the longest dimension of a particle to the shortest dimension of the particle) of greater than 3:1, such as greater than 10:1.

The graphenic carbon particles used in the compositions of the present invention have relatively low oxygen content. For example, the graphenic carbon particles used in certain embodiments of the compositions of the present invention may, even when having a thickness of no more than 5 or no more than 2 nanometers, have an oxygen content of no more than 2 atomic weight percent, such as no more than 1.5 or 1 atomic weight percent, or no more than 0.6 atomic weight, such as about 0.5 atomic weight percent. The oxygen content of the graphenic carbon particles can be determined using X-ray Photoelectron Spectroscopy, such as is described in D. R. Dreyer et al., Chem. Soc. Rev. 39, 228-240 (2010).

In certain embodiments, the graphenic carbon particles used in the compositions of the present invention have a B.E.T. specific surface area of at least 50 square meters per gram, such as 70 to 1000 square meters per gram, or, in some cases, 200 to 1000 square meters per grams or 200 to 400 square meters per gram. As used herein, the term "B.E.T. specific surface area" refers to a specific surface area determined by nitrogen adsorption according to the ASTMD 3663-78 standard based on the Brunauer-Emmett-Teller method described in the periodical "The Journal of the American Chemical Society", 60, 309 (1938).

In certain embodiments, the graphenic carbon particles used in the compositions of the present invention have a Raman spectroscopy 2D/G peak ratio of at least 1.1, for example, at least 1.2 or 1.3. As used herein, the term "2D/G peak ratio" refers to the ratio of the intensity of the 2D peak at 2692 $cm^{-1}$ to the intensity of the G peak at 1,580 $cm^{-1}$.

In certain embodiments, the graphenic carbon particles used in the compositions of the present invention have a relatively low bulk density. For example, the graphenic carbon particles used in certain embodiments of the present invention are characterized by having a bulk density (tap density) of less than 0.2 $g/cm^3$, such as no more than 0.1 $g/cm^3$. For the purposes of the present invention, the bulk density of the graphenic carbon particles is determined by placing 0.4 grams of the graphenic carbon particles in a glass measuring cylinder having a readable scale. The cylinder is raised approximately one-inch and tapped 100 times, by striking the base of the cylinder onto a hard surface, to allow the graphenic carbon particles to settle within the cylinder. The volume of the particles is then measured, and the bulk density is calculated by dividing 0.4 grams by the measured volume, wherein the bulk density is expressed in terms of $g/cm^3$.

In certain embodiments, the graphenic carbon particles used in the compositions of the present invention have a compressed density and a percent densification that is less than the compressed density and percent densification of graphite powder and certain types of substantially flat graphenic carbon particles. Lower compressed density and lower percent densification are each currently believed to contribute to better dispersion and/or rheological properties than graphenic carbon particles exhibiting higher compressed density and higher percent densification. In certain embodiments, the compressed density of the graphenic carbon particles is 0.9 or less, such as less than 0.8, less than 0.7, such as from 0.6 to 0.7. In certain embodiments, the percent densification of the graphenic carbon particles is less than 40%, such as less than 30%, such as from 25 to 30%.

For purposes of the present invention, the compressed density of graphenic carbon particles is calculated from a measured thickness of a given mass of the particles after compression. Specifically, the measured thickness is determined by subjecting 0.1 grams of the graphenic carbon particles to cold press under 15,000 pound of force in a 1.3 centimeter die for 45 minutes, wherein the contact pressure is 500 MPa. The compressed density of the graphenic carbon particles is then calculated from this measured thickness according to the following equation:

$$\text{Compressed Density (g/cm}^3) = \frac{0.1 \text{ grams}}{\Pi*(1.3\text{cm}/2)^2*(\text{measured thickness in cm})}$$

The percent densification of the graphenic carbon particles is then determined as the ratio of the calculated compressed density of the graphenic carbon particles, as determined above, to 2.2 $g/cm^3$, which is the density of graphite.

In certain embodiments, the graphenic carbon particles have a measured bulk liquid conductivity of at least 100 microSiemens, such as at least 120 microSiemens, such as at least 140 microSiemens immediately after mixing and at later points in time, such as at 10 minutes, or 20 minutes, or 30 minutes, or 40 minutes. For the purposes of the present invention, the bulk liquid conductivity of the graphenic carbon particles is determined as follows. First, a sample comprising a 0.5% solution of graphenic carbon particles in butyl cellosolve is sonicated for 30 minutes with a bath sonicator. Immediately following sonication, the sample is placed in a standard calibrated electrolytic conductivity cell (K=1). A Fisher Scientific AB 30 conductivity meter is introduced to the sample to measure the conductivity of the sample. The conductivity is plotted over the course of about 40 minutes.

In accordance with certain embodiments, percolation, defined as long range interconnectivity, occurs between the conductive graphenic carbon particles. Such percolation may reduce the resistivity of the formulations. The conductive graphenic particles may occupy a minimum volume within the composite matrix such that the particles form a continuous, or nearly continuous, network. In such a case, the aspect ratios of the graphenic carbon particles may affect the minimum volume required for percolation. Furthermore, the surface energy of the graphenic carbon particles may be the same or similar to the surface energy of the elastomeric rubber. Otherwise, the particles may tend to flocculate or demix as they are processed.

The graphenic carbon particles utilized in the compositions of the present invention can be made, for example, by thermal processes. In accordance with embodiments of the invention, the graphenic carbon particles are produced from carbon-containing precursor materials that are heated to high temperatures in a thermal zone. For example, the graphenic carbon particles may be produced by the systems and methods disclosed in United States Patent Application Serial Nos. 13/249,315 and 13/309,894.

In certain embodiments, the graphenic carbon particles may be made by using the apparatus and method described in United States Patent Application Serial No. 13/249,315 at [0022] to [0048], in which (i) one or more hydrocarbon precursor materials capable of forming a two-carbon fragment species (such as n-propanol, ethane, ethylene, acetylene, vinyl chloride, 1,2-dichloroethane, allyl alcohol, propionaldehyde, and/or vinyl bromide) is introduced into a thermal zone (such as a plasma); and (ii) the hydrocarbon is heated in the thermal zone to a temperature of at least 1,000°C to form the graphenic carbon particles. In other embodiments, the graphenic carbon particles may be made by using the apparatus and method described in United States Patent Application Serial No. 13/309,894 at [0015] to [0042], in which (i) a methane precursor material (such as a material comprising at least 50 percent methane, or, in some cases, gaseous or liquid methane of at least 95 or 99 percent purity or higher) is introduced into a thermal zone (such as a plasma); and (ii) the methane precursor is heated in the thermal zone to form the graphenic carbon particles. Such methods can produce graphenic carbon particles having at least some, in some cases all, of the characteristics described above.

During production of the graphenic carbon particles by the methods described above, a carbon-containing precursor is provided as a feed material that may be contacted with an inert carrier gas. The carbon-containing precursor material may be heated in a thermal zone, for example, by a plasma system. In certain embodiments, the precursor material is heated to a temperature ranging from 1,000°C to 20,000°C, such as 1,200°C to 10,000°C. For example, the temperature of the thermal zone may range from 1,500 to 8,000°C, such as from 2,000 to 5,000°C. Although the thermal zone may be generated by a plasma system, it is to be understood that any other suitable heating system may be used to create the thermal zone, such as various types of furnaces including electrically heated tube furnaces and the like.

The gaseous stream may be contacted with one or more quench streams that are injected into the plasma chamber through at least one quench stream injection port. The quench stream may cool the gaseous stream to facilitate the formation or control the particle size or morphology of the graphenic carbon particles. In certain embodiments of the invention, after contacting the gaseous product stream with the quench streams, the ultrafine particles may be passed through a converging member. After the graphenic carbon particles exit the plasma system, they may be collected. Any suitable means may be used to separate the graphenic carbon particles from the gas flow, such as, for example, a bag filter, cyclone separator or deposition on a substrate.

Without being bound by any theory, it is currently believed that the foregoing methods of manufacturing graphenic carbon particles are particularly suitable for producing graphenic carbon particles having relatively low thickness and relatively high aspect ratio in combination with relatively low oxygen content, as described above. Moreover, such methods are currently believed to produce a substantial amount of graphenic carbon particles having a substantially curved, curled, creased or buckled morphology (referred to herein as a "3D" morphology), as opposed to producing predominantly particles having a substantially two-dimensional (or flat) morphology. This characteristic is believed to be reflected in the previously described compressed density characteristics and is believed to be beneficial in the present invention because, it is currently believed, when a significant portion of the graphenic carbon particles have a 3D morphology, "edge to edge" and "edge-to-face" contact between graphenic carbon particles within the composition may be promoted. This is thought to be because particles having a 3D morphology are less likely to be aggregated in the composition (due to lower Van der Waals forces) than particles having a two-dimensional morphology. Moreover, it is currently believed that even in the case of "face to face" contact between the particles having a 3D morphology, since the particles may have more than one facial plane, the entire particle surface is not engaged in a single "face to face" interaction with another single particle, but instead can participate in interactions with other particles, including other "face to face" interactions, in other planes. As a result, graphenic carbon particles having a 3D morphology are currently thought to provide the best conductive pathway in the present compositions and are currently thought to be useful for obtaining electrical conductivity characteristics sought by the present invention, particularly when the graphenic carbon particles are present in the composition in the relatively low amounts described below.

In certain embodiments, the graphenic carbon particles are present in the rubber formulations in an amount of at least 0.1 weight percent, such as least 0.5 weight percent, or, in some cases, at least 1 weight percent. In certain embodiments, the graphenic carbon particles are present in the composition in an amount of no more than 15 weight percent, such as no more than 10 weight percent, or, in some cases, no more than 5 weight percent, based on the weight of all non-volatile components of the composition.

In certain embodiments, the base rubber composition of the tire tread or other formulations comprise synthetic rubber, natural rubber and mixes thereof. In certain embodiments, the base rubber composition comprises styrene butadiene co-polymer, polybutadiene, halobutyl and/or natural rubber (polyisoprenes). For use in tire treads, the base rubber composition typically comprises from 30 to 70 weight percent of the overall tire tread formulation, for example, from 34 to 54 weight percent.

In certain embodiments, the rubber formulation comprises a curable rubber. As used herein, the term "curable rubber" means both natural rubber and its various raw and reclaimed forms as well as various synthetic rubbers. For example, the curable rubber can include styrene/butadiene rubber (SBR), butadiene rubber (BR), natural rubber, any other known type of organic rubber, and combinations thereof. As used herein, the terms "rubber", "elastomer" and "rubbery elastomer" can be used interchangeably, unless indicated otherwise. The terms "rubber composition", "compounded rubber" and "rubber compound" can be used interchangeably to refer to rubber which has been blended or mixed with various ingredients and materials, and such terms are well-known to those having skill in the rubber mixing or rubber compounding art.

In addition to the graphenic carbon particles in the amounts described above, the tire tread formulations in certain embodiments also comprise filler particles. Suitable fillers for use in the rubber formulations of the present invention can include a wide variety of materials known to one having ordinary skill in the art. Non-limiting examples can include inorganic oxides such as but not limited to inorganic particulate or amorphous solid materials which possess either oxygen (chemisorbed or covalently bonded) or hydroxyl (bound or free) at an exposed surface such as but not limited to oxides of the metals in Periods 2, 3, 4, 5 and 6 of Groups Ib, IIb, IIIa, IIIb, IVa, IVb (except carbon), Va, VIa, VIIa and VIII of the Periodic Table of the Elements in Advanced Inorganic Chemistry: A Comprehensive Text by F. Albert Cotton et al., Fourth Edition, John Wiley and Sons, 1980. Non-limiting examples of inorganic oxides for use in the present invention can include precipitated silica, colloidal silica, silica gel, aluminum silicates, alumina, and mixtures thereof. Suitable metal silicates can include a wide variety of materials known in the art. Non-limiting examples can include but are not limited to alumina, lithium, sodium, potassium silicate, and mixtures thereof.

In certain embodiments, the filler particles comprise silica in typical amounts of from 1 to 50 weight percent, for example, from 28 to 44 weight percent. In certain embodiments, it is desirable to maximize the amount of silica present in the formulation in order to improve traction and fuel efficiency performance. For example, it may be desirable to add silica in amounts greater than 30 weight percent, for example, greater than 40 weight percent.

In certain embodiments, the silica can be precipitated silica, colloidal silica and mixtures thereof. The silica can have an average ultimate particle size of less than 0.1 micron, or from 0.01 to 0.05 micron, or from 0.015 to 0.02 micron, as measured by electron microscope. In further alternate non-limiting embodiments, the silica can have a surface area of from 25 to 1000 or from 75 to 250 or from 100 to 200 square meters per gram. The surface area can be measured using conventional techniques known in the art. As used herein, the surface area is determined by the Brunauer, Emmett, and Teller (BET) method according to ASTM D1993-91. The BET surface area can be determined by fitting five relative-pressure points from a nitrogen sorption isotherm measurement made with a Micromeritics TriStar 3000.TM. instrument. A FlowPrep-060™ station provides heat and a continuous gas flow to prepare samples for analysis. Prior to nitrogen sorption, the silica samples are dried by heating to a temperature of 160°C in flowing nitrogen (P5 grade) for at least one (1) hour.

The silica filler for use in the present invention can be prepared using a variety of methods known to those having ordinary skill in the art. For example, the silica may be produced by the methods disclosed in U.S. Patent Application Serial No. 11/103,123. In a non-limiting embodiment, silica for use as untreated filler can be prepared by combining an aqueous solution of soluble metal silicate with acid to form a silica slurry. The silica slurry can be optionally aged and acid or base can be added to the optional aged silica slurry. The silica slurry can be filtered, optionally washed, and dried using convention techniques known to a skilled artisan.

In accordance with certain embodiments of the invention, the relative amounts of graphenic carbon particles and silica are controlled such that the amount of silica is maximized for improved performance characteristics, while the amount of graphenic carbon particles is minimized to an amount that provides sufficient static dissipation. For example, the amount of silica may be greater than 30 weight percent, or greater than 40 weight percent, while the amount of graphenic carbon particles may be less than 10 or 5 weight percent, or less than 2 or 1 weight percent. In certain embodiments, the weight ratio of silica particles to graphenic carbon particles is greater than 2:1 or 3:1, for example, greater than 4:1, 5:1 or 6:1. In particular embodiments, the weight ratio may be greater than 8:1 or 10:1.

In a complex system of the present invention where both graphenic carbon particles and silica particles are present in the elastomeric rubber matrix, the conductive graphenic carbon particles may form a continuous, or nearly continuous, network despite the presence of insulating silica particles in the relatively large amounts described above.

In accordance with certain embodiments, the rubber formulations have surface resistivities of less than $10^{10}$ $\Omega$/sq, for example, less than $10^9$ $\Omega$/sq, or less than $10^7$ $\Omega$/sq.

The formulations of the present invention may be made by combining the graphenic carbon particles and/or filler particles with emulsion and/or solution polymers, e.g., organic rubber comprising solution styrene/butadiene (SBR), polybutadiene rubber or a mixture thereof, to form a master batch. Curable rubbers for use in the master batch can vary widely and are well known to the skilled artisan and can include vulcanizable and sulfur-curable rubbers. In a non-limiting embodiment, curable rubbers can include those used for mechanical rubber goods and tires. A non-limiting example of a master batch can comprise a combination of organic rubber, water-immiscible solvent, treated filler and, optionally, processing oil. Such a product can be supplied by a rubber producer to a tire manufacturer. A benefit to a tire manufacturer using a master batch can be that the graphenic carbon particles and/or silica particles are substantially uniformly dispersed in the rubber, which can result in substantially reducing or minimizing the mixing time to produce the compounded rubber.

In a non-limiting embodiment, the master batch can contain from 10 to 150 parts of graphenic carbon particles and/or silica particles per 100 parts of rubber (PHR).

The graphenic carbon particles and/or silica particles can be mixed with an uncured rubbery elastomer used to prepare the vulcanizable rubber composition by conventional means such as in a Banbury mixer or on a rubber mill at temperatures from 100°F and 392°F (38°C-200°C). Non-limiting examples of other conventional rubber additives present in the rubber composition can include conventional sulfur or peroxide cure systems. In alternate non-limiting embodiments, the sulfur-cure system can include from 0.5 to 5 parts sulfur, from 2 to 5 parts zinc oxide and from 0.5 to 5 parts accelerator. In further alternate non-limiting embodiments, the peroxide-cure system can include from 1 to 4 parts of a peroxide such as dicumyl peroxide.

Non-limiting examples of conventional rubber additives can include clays, talc, carbon black, and the like, oils, plasticizers, accelerators, antioxidants, heat stabilizers, light stabilizers, zone stabilizers, organic acids, such as for example stearic acid, benzoic acid, or salicylic acid, other activators, extenders and coloring pigments. The compounding recipe selected will vary with the particular vulcanizate prepared. Such recipes are well known to those skilled in the rubber compounding art. In a non-limiting embodiment, a benefit of the use of silica particles of the present invention when the coupling material is mercaptoorganometallic compound(s) can be the stability at elevated temperatures of a rubber compound containing such silica particles, and essentially the absence of curing of a rubber compounded therewith at temperatures up to at least 200°C when mixed for at least one half minute or up to 60 minutes.

In alternate non-limiting embodiments, the compounding process can be performed batchwise or continuously. In a further non-limiting embodiment, the rubber composition and at least a portion of the graphenic carbon particles and/or silica particles can be continuously fed into an initial portion of a mixing path to produce a blend and the blend can be continuously fed into a second portion of the mixing path.

The following examples are intended to illustrate certain aspects of the present invention, and are not intended to limit the scope of the invention.

Examples

A series of tire tread compounds containing varying amounts of conductive additives were fabricated and evaluated for surface resistivity. A reinforcing network of highly dispersible silica was present in amounts ranging from 47 to 70 parts per 100 rubber (PHR). The conductive particle additives included graphenic carbon particles produced in accordance with embodiments of the present invention, commercially available graphene from XG Sciences, graphite, exfoliated graphite, antimony tin oxide, nickel coated graphite, and polypyrrole coated silica. The graphenic carbon particles were produced by the method disclosed in United States Patent Application Serial No. 13/309,894. The components listed in Table 1 were blended and cured using equipment and techniques well known in the tire tread formulation art. Styrene butadiene rubber and polybutadiene rubber were mixed with the conductive additives, fillers, processing aids, antioxidants and part of the cure package in the first pass to form a master batch. The components were mixed for 7 minutes or until the compound reached 160°C. In the second pass, the master batch was then fed back into the mixer and processed for an additional 10 minutes at 160°C. In the third and final mixing pass, the remaining curatives and accelerators are added to the masterbatch and mixed for 2.5 minutes at 108°C.

Table 1

| Tire Tread Compositions | | | | | | | |
|---|---|---|---|---|---|---|---|
| Composition/Ingredients (PHR) | A | B | C | D | E | F | Control |
| Pass 1 | | | | | | | |
| Budene 1207[1] | 30.01 | 30.01 | 30.01 | 30.01 | 30.01 | 30.01 | 30.01 |
| VSL - 5025-2 HM[2] | 96.28 | 96.26 | 96.28 | 96.28 | 96.28 | 96.28 | 96.28 |
| Si -266[3] | 7.53 | 11.00 | 11.00 | 11.00 | 11.00 | 7.53 | 11.00 |
| Precipitated Silica | 47.09 | 49.02 | 49.02 | -- | 70.00 | 47.09 | 70.00 |
| Conductive Particles | 22.92 | 21.01 | 24.89 | 70.00 | 23.64 | 22.92 | 0.00 |
| Microsere 5816A | 2.00 | 2.00 | 2.00 | 2.00 | 2.00 | 2.00 | 2.00 |
| Nocheck 4757A | 1.00 | 1.00 | 1.00 | 1.00 | 1.00 | 1.00 | 1.00 |
| TMQ | 1.00 | 1.00 | 1.00 | 1.00 | 1.00 | 1.00 | 1.00 |
| Santoflex 13 | 2.00 | 2.00 | 2.00 | 2.00 | 2.00 | 2.00 | 2.00 |

(continued)

| Tire Tread Compositions | | | | | | | |
|---|---|---|---|---|---|---|---|
| Composition/Ingredients (PHR) | A | B | C | D | E | F | Control |
| Stearic Acid | 2.00 | 2.00 | 2.00 | 2.00 | 2.00 | 2.00 | 2.00 |
| Tufflo 100 | 5.00 | 5.00 | 5.00 | 5.00 | 5.00 | 5.00 | 5.00 |
| Zinc Oxide (720C) | 3.50 | 3.50 | 3.50 | 3.50 | 3.50 | 3.50 | 3.50 |
| Pass 2 | | | | | | | |
| Master Batch | | | | | | | |
| Pass 3 | | | | | | | |
| Master Batch | | | | | | | |
| RM Sulfur [4] | 1.70 | 1.70 | 1.70 | 1.70 | 1.70 | 1.70 | 1.70 |
| Santocure CBS [5] | 2.00 | 2.00 | 2.00 | 2.00 | 2.00 | 2.00 | 2.00 |
| diphenyl guanidine (DPG) | 2.00 | 2.00 | 2.00 | 2.00 | 2.00 | 2.00 | 2.00 |

A - Graphenic Carbon Particles
B - Nickel Coated Graphite
C - Antimony Tin Oxide
D - Polypyrrole Coated Silica
E -Asbury Carbon Graphites (3725, M850, 4014, 3775, 4821, 230U)
F - XG Sciences Graphene (C-750, C-300, M-25, M-5)
[1] Polybutadiene rubber available from The Goodyear Tire and Rubber Company
[2] Styrene butadiene rubber available from Lanxess
[3] Bis(triethoxysilylpropyl)polysulfide available from Evonik
[4] Commercially available rubber-makers sulfur
[5] N-cyclohexylbenzo-thiazole-2-sulfenamide available from Flexsys

The surface resistivities of the finished, cured rubber materials were measured according to the following procedure: Dr. Thiedig Milli-To Ohm Meter turned on and allowed to equilibrate for 0.5 hour prior to experimental sampling; rubber sample is placed on insulating plastic slab; 5 lb concentric circle grounded electrode placed on rubber sample with gentle pressure to ensure even contact; electrode voltage is applied, surface resistivity measured using the lowest possible setting of 10,100 or 500 volts; and surface resistivity is determined by $10\times$ the on-screen reading with units of $\Omega$ or $\Omega$/sq.

The surface resistivity results are shown in Table 2. Materials in which the surface resistivity is in the range of from $10^6$ to $10^9$ or $10^{10}$ are said to be static dissipative. For silica filled tread composites, it may be desirable for the percolation threshold of the conductive filler to be at a minimum in both weight and volume percent. Of the materials tested, the graphenic carbon particles of the present invention were the only particles that showed static dissipative properties at low loadings (5 volume %) in the presence of silica. In addition to the electrical properties of the finished rubber goods, the graphenic carbon particles exhibited uniquely improved mixing properties.

Table 2

| Surface Resistivities of Rubber Formulations | | | |
|---|---|---|---|
| Composition | Weight % | Volume % | Surface Resistivity ($\Omega$/sq) |
| A Graphenic Carbon Particles | 10.14 | 5.4 | $8.0\times10^6$ |
| B Nickel Coated Graphite | 9.15 | 7.15 | $1.4\times10^{14}$ |
| C Antimony Tin Oxide | 9.15 | 3.09 | $5.9\times10^{13}$ |
| D Polypyrrole Coated Silica | 30.5 | 16.83 | $5.0\times10^{13}$ |
| E Asbury Carbon Samples | 9.34 | 5.04 | $10^{14}$ |
| F XG Sciences Graphenes | 10.14 | 5.4 | $10^{13}$ |

(continued)

| Surface Resistivities of Rubber Formulations | | | |
|---|---|---|---|
| Composition | Weight % | Volume % | Surface Resistivity ($\Omega$/sq) |
| Control - Silica with no conductive particles | --- | --- | $5.0\times10^{13}$ |

In certain embodiments, it is desirable to improve the dispersion of silica in the rubber mixture by breaking down large silica agglomerates that may be present into smaller or submicron particles. The quality of silica dispersion may be determined using a piece of equipment called a dispergrader. When examining rubber samples using this device, the amount of white area should be at a minimum. The silica dispersion may be important for consistent performance, wear, obtaining good reinforcement, and for limiting failures such as crack propagation. Thus, fillers that significantly reduce silica dispersion at low loadings may not be acceptable. Normalized silica dispersions for tread compounds prepared using highly dispersible silica and various types of conductive particles and silica are shown in Table 3.

Table 3

| Dispersion of Conductive Particles (Normalized to HDS) | | | |
|---|---|---|---|
| Composition | PHR Conductive Particles | PHR Silica | Normalized Dispersion |
| Highly Dispersible Silica and Graphenic Carbon Particles | 23.0 | 47.0 | 1.03 |
| Highly Dispersible Silica and XG Sciences C-750 | 23.0 | 47.0 | 0.55 |
| Highly Dispersible Silica and XG Sciences C-300 | 23.0 | 47.0 | 0.65 |
| Highly Dispersible Silica and XG Sciences M-25 | 23.0 | 47.0 | VERY POOR |
| Highly Dispersible Silica and XG Sciences M-5 | 23.0 | 47.0 | 0.36 |
| Highly Dispersible Silica and Asbury Graphite 3775 | 23.0 graphite | 47.0 | 0.82 |
| Highly Dispersible Silica | 0 | 70 | 1.00 |

In certain embodiments, the graphenic carbon particles may provide improved reinforcing properties due to their high specific surface areas relative to the volume they occupy. Tire tread compounds fabricated with graphenic carbon particles and silica particles may exhibit increased tensile strength and improvements in traction as defined by the tan $\delta$ at 0°C. The rolling resistance is increased while the abrasive wear may remain unchanged. These properties are shown in Table 4.

Table 4

| Tread Properties | | | | | |
|---|---|---|---|---|---|
| Composition | PHR Graphene | PHR Silica | Tensile Strength (MPa) | Traction (tan $\delta$ @ 0) | DIN Abrasion |
| Highly Dispersible Silica and Graphenic Carbon Particles | 23.0 | 47.0 | 15.7 | 0.314 | 159.0 |
| Highly Dispersible Silica | 0 | 70 | 14.0 | 0.223 | 159.0 |

The combination of low percolation threshold, improved tensile strength and excellent silica dispersion achieved by the use of graphenic carbon particles in accordance with the present invention make the formulations very useful in tire treads.

In certain embodiments, by pre-dispersing the graphenic carbon particles into a compatible resin, the resistivity of a tread compound can be reduced at lower graphenic carbon particle loadings, as shown in Table 5. In this example, the graphenic carbon particles are predispersed in a sulfur containing resin commercially known as Thioplast.

Table 5

| Surface Resistivities of Treads Made with Pre-Dispersed Graphene | | | |
|---|---|---|---|
| Composition | Weight % | Volume % | Surface Resistivity ($\Omega$/sq) |
| Graphenic Carbon Particles | 10.14 | 5.4 | $8.0 \times 10^6$ |
| Graphenic Carbon Particles in Thioplast Dispersion | 4.8 | 2.5 | $2.7 \times 10^{10}$ |
| XG Sciences Graphenes | 10.14 | 5.4 | $10^{13}$ |
| Control - silica with no conductive particles | --- | --- | $5.0 \times 10^{13}$ |

A criteria for assessing the performance of conductive filler particles in systems with non-conductive fillers may be to assess the resistivity of a rubber sample at different insulating filler to conductive filler ratios, e.g., the volume or weight of silica to graphenic carbon particles. As the ratio of the non-conductive filler to conductive filler decreases, percolation may be observed at lower loadings of conductive filler. Table 6 illustrates improved surface resistivity of a sample of the present invention containing graphenic carbon particles at a relatively high volume ratio of silica to graphenic carbon particles, in comparison with another sample having the same volume ratio but different conductive particles.

Table 6

| Composition | Volume % Silica | Volume % Graphenic Carbon Particles | Volume Ratio | Surface Resistivity ($\Omega$/sq) | Comment |
|---|---|---|---|---|---|
| Graphenic Carbon Particles | 11.6 | 5.4 | 2.15:1 | $8.0 \times 10^6$ | Percolation |
| XG Sciences Graphenes | 11.6 | 5.4 | 2.15:1 | $10^{13}$ | No percolation |

For purposes of this detailed description, it is to be understood that the invention may assume various alternative variations and step sequences, except where expressly specified to the contrary. Moreover, unless otherwise indicated, all numbers expressing quantities used in the specification and claims are to be understood as being modified in all instances by the term "about". Accordingly, unless indicated to the contrary, the numerical parameters set forth in the following specification and attached claims are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variation found in their respective testing measurements.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

In this application, the use of the singular includes the plural and plural encompasses singular, unless specifically stated otherwise. In addition, in this application, the use of "or" means "and/or" unless specifically stated otherwise, even though "and/or" may be explicitly used in certain instances.

**Claims**

1. A rubber formulation comprising:

   a base rubber composition;
   from 0.1 to 20 weight percent graphenic carbon particles; and
   from 1 to 50 weight percent filler particles, wherein the graphenic carbon particles have an oxygen content of less than 2 atomic weight percent and the rubber formulation has a surface resistivity of less than $10^{10}$ $\Omega$/sq.

**2.** The rubber formulation of Claim 1, wherein the rubber formulation comprises a tire tread formulation.

**3.** The rubber formulation of Claim 1, wherein the graphenic carbon particles comprise less than 10 weight percent of the formulation, preferably less than 5 weight percent of the formulation.

**4.** The rubber formulation of Claim 1, wherein the graphenic carbon particles have a bulk liquid conductivity of at least 100 microSiemens.

**5.** The rubber formulation of Claim 1, wherein the graphenic carbon particles have a bulk density of less than 0.2 g/cm$^3$.

**6.** The rubber formulation of Claim 1, wherein the graphenic carbon particles have a compressed density of less than 0.9.

**7.** The rubber formulation of Claim 1, wherein the filler particles comprise silica.

**8.** The rubber formulation of Claim 7, wherein the silica comprises from 28 to 44 weight percent of the formulation.

**9.** The rubber formulation of Claim 7, wherein the silica comprises precipitated silica.

**10.** The rubber formulation of Claim 1, wherein the base rubber composition comprises styrene/butadiene rubber, butadiene rubber, natural rubber and/or functionalized derivatives thereof and preferably further comprises at least one additive selected from processing oils, antioxidants, curatives and metal oxides.

**11.** The rubber formulation of Claim 1, wherein the formulation comprises less than 10 weight percent carbon black, and preferably is substantially free of carbon black.

**12.** A method of making a rubber formulation comprising:

mixing graphenic carbon particles and filler particles with a base rubber composition; and
curing the mixture, wherein the graphenic carbon particles have an oxygen content of less than 2 atomic weight percent and the cured mixture has a surface resistivity of less than 10$^{10}$ Ω/sq.

**13.** The rubber formulation of claim 1 or the method of Claim 12, wherein the graphenic carbon particles comprise less than 10 weight percent of the formulation, and the filler particles comprise silica in an amount greater than 28 weight percent of the formulation.

**14.** The rubber formulation of claim 7 or the method of Claim 13, wherein the silica and the graphenic carbon particles are present in the formulation in a weight ratio of greater than 4:1.

**Patentansprüche**

**1.** Eine Kautschukformulierung umfassend:

eine Kautschukgrundzusammensetzung;

0,1 bis 20 Gew.-% graphenartiger Kohlenstoffteilchen; und
1 bis 50 Gew.-% Füllstoffteilchen,

wobei die graphenartigen Kohlenstoffteilchen einen Sauerstoffgehalt von weniger als 2 At.-Gew.-% haben und die Kautschukformulierung einen Oberflächenwiderstand von weniger als 10$^{10}$ Ω/□ hat.

**2.** Die Kautschukformulierung gemäß Anspruch 1, wobei die Kautschukformulierung eine Reifenprofilformulierung umfasst.

**3.** Die Kautschukformulierung gemäß Anspruch 1, wobei die graphenartigen Kohlenstoffteilchen weniger als 10 Gew.-% der Formulierung ausmachen, vorzugsweise weniger als 5 Gew.-% der Formulierung.

**4.** Die Kautschukformulierung gemäß Anspruch 1, wobei die graphenartigen Kohlenstoffteilchen eine Gesamtleitfähigkeit der Flüssigkeit von mindestens 100 μS haben.

**5.** Die Kautschukformulierung gemäß Anspruch 1, wobei die graphenartigen Kohlenstoffteilchen eine Schüttdichte von weniger als 0,2 g/cm$^3$ haben.

**6.** Die Kautschukformulierung gemäß Anspruch 1, wobei die graphenartigen Kohlenstoffteilchen eine Pressdichte von weniger als 0,9 aufweisen.

**7.** Die Kautschukformulierung gemäß Anspruch 1, wobei die Füllstoffteilchen Siliciumoxid umfassen.

**8.** Die Kautschukformulierung gemäß Anspruch 7, wobei das Siliciumoxid 28 bis 44 Gew.-% der Formulierung ausmacht.

**9.** Die Zusammensetzung gemäß Anspruch 7, wobei das Siliciumoxid Fällungskieselsäure umfasst.

**10.** Die Kautschukformulierung gemäß Anspruch 1, wobei die Kautschukgrundzusammensetzung Styrol/Butadienkautschuk, Butadienkautschuk, natürlichen Kautschuk und/oder funktionalisierte Derivate derselben enthält und vorzugsweise weiterhin mindestens ein Additiv ausgewählt aus den Gruppen bestehend aus Prozessölen, Antioxidantien, Härtern und Metalloxiden umfasst.

**11.** Die Kautschukformulierung gemäß Anspruch 1, wobei die Formulierung weniger als 10 Gew.-% Ruß enthält und vorzugsweise im Wesentlichen frei von Ruß ist.

**12.** Ein Verfahren zur Herstellung einer Kautschukformulierung umfassend:

Mischen von graphenartigen Kohlenstoffteilchen und Füllstoffteilchen mit einer Kautschukgrundzusammensetzung; und
Härten der Mischung, wobei die graphenartigen Kohlenstoffteilchen einen Sauerstoffgehalt von weniger als 2 At.-Gew.-% aufweisen und die gehärtete Mischung einen Oberflächenwiderstand von weniger $10^{10}$ Ω/□ hat.

**13.** Die Kautschukformulierung gemäß Anspruch 1 oder das Verfahren gemäß Anspruch 12, wobei die graphenartigen Kohlenstoffteilchen weniger als 10 Gew.-% der Formulierung ausmachen und die Füllstoffteilchen Siliciumoxid in einer Menge von mehr als 28 Gew.-% der Formulierung umfassen.

**14.** Die Kautschukformulierung gemäß Anspruch 7 oder das Verfahren gemäß Anspruch 13, wobei das Siliciumoxid und die graphenartigen Kohlenstoffteilchen in der Formulierung in einem Gewichtsverhältnis von größer als 4:1 vorhanden sind.

**Revendications**

**1.** Formulation de caoutchouc comprenant :

- une composition de caoutchouc de base,
- de 0,1 à 20 % en poids de particules de carbone sous la forme graphène,
- et de 1 à 50 % en poids de particules de charge,

dans laquelle les particules de carbone sous forme graphène présentent une teneur en oxygène inférieure à 2 % en atomes,
et laquelle formulation de caoutchouc présente une résistivité surfacique inférieure à $10^{10}$ Ω/□.

**2.** Formulation de caoutchouc conforme à la revendication 1, laquelle formulation de caoutchouc comprend une formulation pour bande de roulement de pneumatique.

**3.** Formulation de caoutchouc conforme à la revendication 1, dans laquelle les particules de carbone sous forme graphène représentent moins de 10 % du poids de la formulation, et de préférence, moins de 5 % du poids de la formulation.

**4.** Formulation de caoutchouc conforme à la revendication 1, dans laquelle les particules de carbone sous forme graphène donnent une conductivité au sein d'un liquide d'au moins 100 microsiemens

**5.** Formulation de caoutchouc conforme à la revendication 1, dans laquelle les particules de carbone sous forme graphène présentent une masse volumique apparente inférieure à 0,2 g/cm$^3$.

**6.** Formulation de caoutchouc conforme à la revendication 1, dans laquelle les particules de carbone sous forme graphène présentent, à l'état comprimé, une densité inférieure à 0,9.

**7.** Formulation de caoutchouc conforme à la revendication 1, dans laquelle les particules de charge comprennent de la silice.

**8.** Formulation de caoutchouc conforme à la revendication 7, dans laquelle la silice représente de 28 à 44 % du poids de la formulation.

**9.** Formulation de caoutchouc conforme à la revendication 7, dans laquelle la silice comprend de la silice précipitée.

**10.** Formulation de caoutchouc conforme à la revendication 1, dans laquelle la composition de caoutchouc de base comprend un caoutchouc de styrène/butadiène, un caoutchouc de butadiène, un caoutchouc naturel et/ou de leurs dérivés fonctionnalisés, et comprend de préférence au moins un adjuvant choisi parmi les huiles plastifiantes, anti-oxydants, durcisseurs et oxydes de métaux.

**11.** Formulation de caoutchouc conforme à la revendication 1, laquelle formulation comprend moins de 10 % en poids de noir de carbone, et de préférence, est pratiquement exempte de noir de carbone.

**12.** Procédé de fabrication d'une formulation de caoutchouc, comportant les étapes suivantes :

- mélanger des particules de carbone sous forme graphène et des particules de charge avec une composition de caoutchouc de base,
- et faire durcir le mélange,

dans lequel les particules de carbone sous forme graphène présentent une teneur en oxygène inférieure à 2 % en atomes, et le mélange durci présente une résistivité surfacique inférieure à $10^{10}$ Ω/□.

**13.** Formulation de caoutchouc conforme à la revendication 1, ou procédé conforme à la revendication 12, dans laquelle ou lequel les particules de carbone sous forme graphène représentent moins de 10 % du poids de la formulation, et les particules de charge comprennent de la silice, en une quantité représentant plus de 28 % du poids de la formulation.

**14.** Formulation de caoutchouc conforme à la revendication 7, ou procédé conforme à la revendication 13, dans laquelle ou lequel la silice et les particules de carbone sous forme graphène se trouvent présentes dans la formulation en un rapport pondéral supérieur à 4/1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20100096918 A
- US 13249315 B
- US 13309894 B
- US 103123 A

**Non-patent literature cited in the description**

- **YONG-GU KANG.** *Kor. J. Meter. Res.,* 2010, vol. 20, 104-110
- **D. R. DREYER et al.** *Chem. Soc. Rev.,* 2010, vol. 39, 228-240
- *The Journal of the American Chemical Society,* 1938, vol. 60, 309
- **F. ALBERT ; COTTON et al.** Advanced Inorganic Chemistry: A Comprehensive Text. John Wiley and Sons, 1980